(19) Europäisches Patentamt
European Patent Office
Office européen des brevets

(11) Publication number: 0 288 320 A2

# (12) EUROPEAN PATENT APPLICATION

(21) Application number: 88303707.9

(22) Date of filing: 25.04.88

(51) Int. Cl.[4]: C 07 C 69/58
C 07 C 67/48, C 07 C 69/48,
C 07 C 69/34

(30) Priority: 23.04.87 IT 2021587

(43) Date of publication of application:
26.10.88 Bulletin 88/43

(84) Designated Contracting States:
BE DE FR GB IT NL SE

(71) Applicant: Raffineria Olii Lubrificanti "R.O.L." S.p.A.
50, Via De Notaris
Milan (IT)

(72) Inventor: Bornengo, Giorgio
8, via Paletta
Novara (IT)

Agnes, Giovanni
22, Via Corridoni
Novara (IT)

Botti, Diego
9, Via Neppiano
Varzi Pavia (IT)

Rolando, Ambrogio
2, Via Torina
Villadeati Alessandria (IT)

(74) Representative: Whalley, Kevin et al
MARKS & CLERK 57/60 Lincoln's Inn Fields
London WC2A 3LS (GB)

## (54) Process for removing soluble compounds of tin from esters of mono- or poly-carboxylic acids.

(57) A process for removing soluble compounds of tin from esters of mono- or poly-carboxylic acids and of mono- or poly-hydric alcohols, useful in the field of oleodynamic fluids, wherein the esters are treated with 5-sulfo-salicylic acid, whereby tin is precipitated as an insoluble compound, and the thus formed precipitate is separated from the purified esters.

EP 0 288 320 A2

**Description**

## "PROCESS FOR REMOVING SOLUBLE COMPOUNDS OF TIN FROM ESTERS OF MONO- OR POLY-CARBOXYLIC ACIDS"

This invention relates to a process for removing soluble compounds of tin from esters of mono- or poly-carboxylic acids and of mono- or poly-hydric alcohols.

It is known that some derivatives of tin, such as stannous oxide and stannous salts of mono- or poly-carboxylic acids, act as catalysts in the synthesis of aliphatic esters, starting from mono- or poly-carboxylic fatty acids and from mono- or poly-hydric alcohols, and it is known that these esters are widely used in industry as oleodynamic fluids, lubricants, cutting fluids, insulating oil, (dielectric oils), drilling muds etc. See for instance European Patent No. EP-A-202 878 and U.S. Patent Nos. US-A-3,637,501; 3,778,454; 3,526,596; and 3,894,959.

A drawback of the above mentioned preparation is that a tin containing catalyst can give rise to compounds soluble in the reaction medium and a fluid containing such soluble compounds exhibits an undesirable capacity to emulsify water. Furthermore such preparation can give rise, by extended use, to insoluble stannous or stannic compounds, which can cause serious drawbacks to the apparatus they are used in; moreover, the toxicity of tin compounds is very well known.

The amount of tin is generally from 300 to 500 ppm, based on the fluid, whereas the end use of the same fluid requires an almost quantitative purification from esters containing tin. This goal, however, cannot be reached by easy and conventional methods; several methods, already used for precipitating tin from different organic liquids, such as for example conversion into sulfides, phosphates, oxides, hydroxides as well as oxalates of tin, did not provide any positive result as to the carboxylic esters to be treated by the present invention. A recent method providing the removal of tin and other metals from oleodynamic fluids consisting of another type of esters (phosphoric esters) by use of salts of ethylenediaminotetracetic acid (see U.S. Patent Nos. US-A-4,205,023 and 4,264,534), proved to be quite unsatisfactory when applied to the carboxylic esters to be treated by the present invention.

We have now found that a specific and quite particular agent, exhibiting kelating properties, allows a quick precipitation of tin contained as a soluble compound in the above mentioned esters, practically quantitatively, and in a readily realizable manner.

The present invention provides a process for removing soluble compounds of tin from esters of mono-or poly-carboxylic acids and of mono- or poly-hydric alcohols, characterized in that said ester is treated with 5-sulfo-salicyclic acid or with an alkaline salt thereof, to precipate tin as an insoluble compound, and in that the thus formed precipitate is separated from the purified ester.

The treatment is preferably carried out from room temperature to 150°C, more preferably at 50-120°C.

The treatment is preferably carried out under an inert gas, in particular nitrogen.

The 5-sulfo-salicylic acid is preferably used in the form of dihydrate.

Generally the invention is concerned with a process for removing soluble compounds of tin from esters of mono- or poly-carboxylic acids and mono- or poly-hydric alcohols and in particular from esters of formula (I):

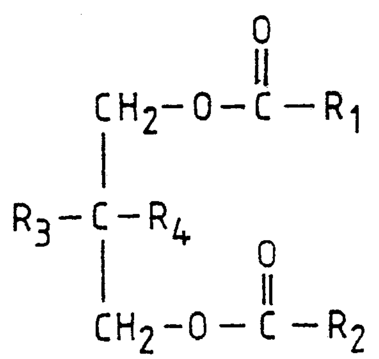

(I)

wherein $R_1$ and $R_2$, which may be the same as or different from each other, are alkyl or alkylene groups containing from 5 to 24 carbon atoms, optionally substituted with carboxylic or hydroxylic groups; and wherein $R_3$ and $R_4$, which may be the same as or different from each other, are selected from hydrogen, groups of the $R_1$ type, groups of formula (II):

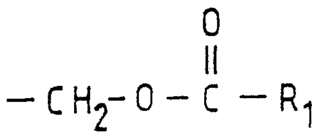

(II)

and (in the case of polymeric esters) groups of formula (III):

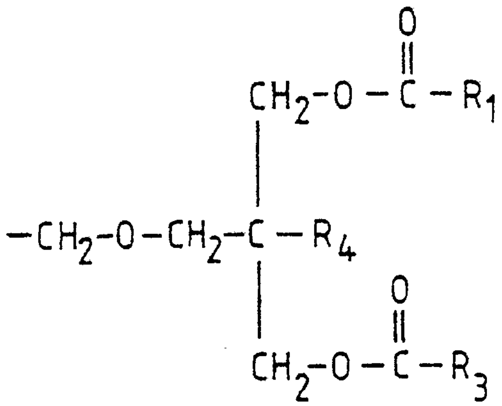

(III)

The process is characterized in that the above mentioned esters are treated with 5-sulfo-salicylic acid, of the formula (IV):

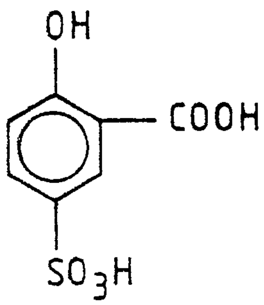

(IV)

or with an alkaline salt thereof (alkali or alkaline earth salt), whereupon tin precipitates as an insoluble compound, and that the thus obtained precipitate is separated from the purified esters by generally used techniques, such as decantation, filtration or centrifugation.

The process is particularly advantageous when applied to the purification of fluids obtained from esterification of polyhydric alcohols, such as glycerol, trimethylol-propane, pentaerythritol, sorbitol, sorbitane, glycols or polyoxyalkylene-glycols, with an acid selected from oleic acid, azelaic acid, sebacic acid, citric acid, fatty acids in general etc. The invention gives rise to surprising results; many other compounds belonging to the class of aromatic compounds and containing carboxylic groups and/or hydroxylic and/or sulfonic groups, such as salicylic acid, naphthalene-2-oxy-3, 6-disulfonic acid, benzoic acid, 2-oxy-3-naphthoic acid or dihydroxybenzene, proved in fact to be quite inactive in precipitating tin. The last traces of 5-sulfo-salicylic acid in the treated fluid do not give rise to any corrosion, and the process of the invention gives rise to an advantageous increase of the demulsifying capacity of the treated fluid.

According to a particularly advantageous embodiment of the invention, an amount of 5-sulfo-salicylic acid (anhydrous or di-hydrate) equal to or higher than the stoichiometric amount (requested by the reaction with the tin derivative) is dissolved into the fluid to be purified, under agitation, extending the reaction time until tin compounds are completely precipitated.

The molar ratio between 5-sulfo-salicylic acid and tin is preferably from 1 to 10, more preferably from 1 to 5, and the temperature of the treatment (preferably under inert gas) is preferably comprised between room temperature and 150° C; an increase of the temperature generally makes the precipitation quicker. Generally a temperature from 50 to 120° C and a reaction time from 10 minutes to 1 hour represent a satisfactory operative range.

The invention will be further described with reference to the following illustrative Examples.

EXAMPLE 1

An oleodynamic fluid, sold under the trade-name "PENTAFLUX" by the Applicants, was obtained by esterifying trimethylolpropane with a mixture of fatty acids (obtained by alkaline hydrolysis of natural oleines) in the presence of stannous oxide; 100g of the above mentioned fluid, containing 0.23 x $10^{-3}$ ion gram of $Sn^{+}$1, corresponding to 274 ppm of tin, as an undesired impurity, were loaded into a 250 $cm^3$ round-bottomed glass flask (provided with mechanical rotating agitator, thermometer and reflux cooler) dipped in a thermostatic oil bath. The temperature was raised to 100° C (under nitrogen) and thereafter 0.06 g (0.27 millimols) of dihydrated 5-sulfo-salicylic acid was added. The agitation was continued for 30 minutes, always keeping the temperature at 100°C; 1 g of diatomaceous earth was added and the whole was filtered on a porous baffle. The determination of tin was carried out by atomic absorption by means of a Perkin Elmer spectrophotometer Mod. 503, equipped with E.D.L. lamp and microwave feeder, and the preparation of the sample was carried out by destroying the organic substance with fuming $H_2SO_4$, and then with concentrated $HNO_3$ and $H_2O_2$ (100 volumes). The quantitative determination was calculated by subtracting from the thus obtained result the amount of tin existing in a blank solution (namely a solution obtained by using the same amounts of reactants but excluding the sample to be examined); the analysis of the filtrate showed the presence of traces of tin lower than the identification limit of the method (about 10 ppm).

EXAMPLE 2 (Comparative)

Example 1 was repeated without adding 5-sulfo-salicylic acid; the analysis of the filtrate showed the presence of about 0.20 x $10^{-3}$ion gram of tin, corresponding to 270 ppm. This means that diatomaceous earth alone is unable to retain the tin present in the fluid.

EXAMPLE 3

A PENTAFLUX fluid was prepared as described in example 1, adding azelaic acid to the mixture of fatty acids deriving from oleines. 100 g of fluid, containing 0.22 x $10^{-3}$ion gram of $Sn^{+}$1, corresponding to 270 ppm of tin, as an undesired impurity, were loaded in to the same round-bottomed flask of example 1. The temperature was raised to 100°C (under nitrogen) and then 0.16 g (0.63 millimols) of 5-sulfo-salicylic acid (di-hydrate) was added. The agitation was continued for 30 minutes, always keeping the temperature at 100°C; 1 g of diatomaceous earth was added and the whole was filtered on a porous baffle. The analysis of the filtrate by atomic absorption showed the presence of traces of tin lower than 20 ppm.

EXAMPLE 4

100 g of the fluid of example 1, containing 0.3 x $10^{-3}$ion gram of $Sn^{+}$1, corresponding to 350 ppm of tin, namely a higher amount (in comparison with preceding examples) were introduced into the same round-bottomed flask of example 1. The temperature was raised to 100° C and thereafter 0.16 g (0.63 millimols) of 5-sulfo-salicylic acid (di-hydrate) was added. The agitation was continued for 30 minutes, always keeping the temperature at 100° C and the precipitate was separated by filtration on paper; the analysis of the filtrate by atomic absorption showed the presence of traces of tin lower than 20 ppm.

EXAMPLES 5 to 15

Example 4 was repeated by working on 50 g of oleodynamic fluid, replacing the 5-sulfo-salicylic acid by other kelating agents and changing the operative conditions as indicated in Table 1; satisfactory results were obtained only and exclusively when 5-sulfo-salicylic acid was used. As will be apparent, Examples 5 to 12 are Comparative Examples.

EXAMPLES 16 to 23

Example 4 was replaced by a series of experimentations carried out in order to define optimum conditions for the purification of the fluid; the results are given in Table II.

TABLE I (**)

| EXAMPLES | KELATING AGENT FORMULA | KELATING AGENT Quantity (mmols) | Kel.mols / Sn mols | T (°C) | Sn final (ppm) (***) |
|---|---|---|---|---|---|
| 5 (*) | 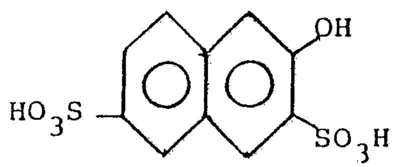 | 1.46 | 10 | 25 | 340 |
| 6 (*) | As above | 1.46 | 10 | 50 | 350 |
| 7 (*) | As above | 1.46 | 10 | 100 | 350 |
| 8 (*) | 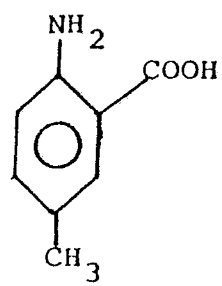 | 1.46 | 10 | 50 | 350 |
| 9 (*) | As above | 1.46 | 10 | 25 | 150 |
| 10 (*) | 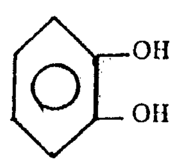 | 1.46 | 10 | 50 | 340 |

TABLE I (**) (follows)

| EXAMPLES | KELATING AGENT FORMULA | Quantity (mols) | Kel.mols / Sn mols | T (°C) | Sn final (ppm) (***) |
|---|---|---|---|---|---|
| 11 (*) | 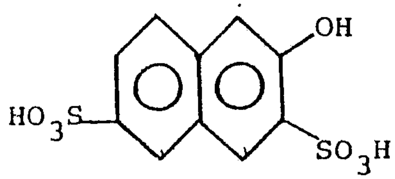 | 0.7 | 5 | 25 | 350 |
| 12 (*) | As above | 1.46 | 10 | 25 | 330 |
| 13 | 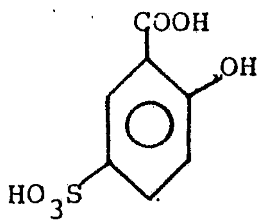 | 0.7 | 5 | 25 | 44 |
| 14 | As above | 0.7 | 5 | 50 | 20 |
| 15 | As above | 1.4 | 10 | 25 | 40 |

(*) Comparative example

(**) Final filtration on paper; reaction time = 3 hours

(***) Starting quantity of tin = 350 ppm

TABLE II (**)

| EX. | Quantity of fluid (g) | Quantity of kelating agent (mmol) | Kel.mols / Sn mols | Reaction time (h) | T (°C) | Final Sn (ppm) (*) |
|---|---|---|---|---|---|---|
| 16 | 50 | 1.4 | 10 | 3 | 50 | 44 |
| 17 | 50 | 0.5 | 3.5 | 3 | 100 | < 20 |
| 18 | 50 | 0.5 | 3.5 | 3 | 100 | < 20 |
| 19 | 50 | 0.5 | 3.5 | 1.5 | 100 | < 20 |
| 20 | 50 | 0.5 | 3.5 | 0.5 | 100 | < 20 |
| 21 | 100 | 0.7 | 2.5 | 0.5 | 100 | 26 |
| 22 | 100 | 0.5 | 1.8 | 0.5 | 100 | 70 |
| 23 | 750 | 10.5 | 5 | 0.5 | 50 | < 20 |

(*) Starting quantity of tin = 340 ppm

(**) Final filtration on paper

EXAMPLE 24

Example 23 was repeated on the fluid of example 3, prepared by adding azelaic acid (di-carboxylic acid) to fatty acids coming from oleines; the above mentioned fluid originally contained 340 ppm of tin. The analysis of the final filtrate (by atomic absorption) showed the presence of traces of tin lower than 20 ppm.

## Claims

1. A process for removing soluble compounds of tin from esters of mono- or poly-carboxylic acids and of mono- or poly-hydric alcohols, characterized in that said ester is treated with 5-sulfo-salicylic acid or with an alkaline salt thereof, to precipate tin as an insoluble compound, and in that the thus formed precipitate is separated from the purified ester.

2. A process as claimed in Claim 1, characterized in that the said treatment is carried out from room temperature to 150° C, preferably at 50-120° C.

3. A process as claimed in Claim 1 or 2 , characterized in that the said treatment is carried out under an inert gas, preferably under nitrogen.

4. A process as claimed in any of Claims 1 to 3, characterized in that the 5-sulfo-salicylic acid is used in the form of dihydrate.

5. A process as claimed in any of Claims 1 to 4, characterized in that the molar ratio:

$$\frac{\text{5-sulfo-salicylic acid}}{\text{Sn present at the starting in the esters}}$$

is from 1 to 10, preferably from 1 to 5.

6. A process as claimed in any of Claims 1 to 5, characterized in that said esters have the formula (I):

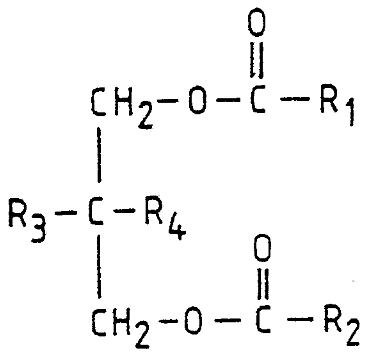

(I)

wherein $R_1$ and $R_2$, which may be the same as or different from each other, are alkly or alkylene groups containing from 5 to 24 arbon atoms, optionally substituted by carboxylic and/or hydroxylic groups; and wherein $R_3$ and $R_4$, which may be the same as or different from each other, are selected from hydrogen, groups of the $R_1$ type, groups of formula (II);

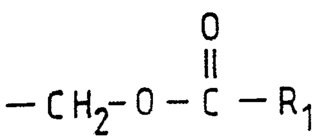

(II)

and (for polymeric esters) groups of formula (III):

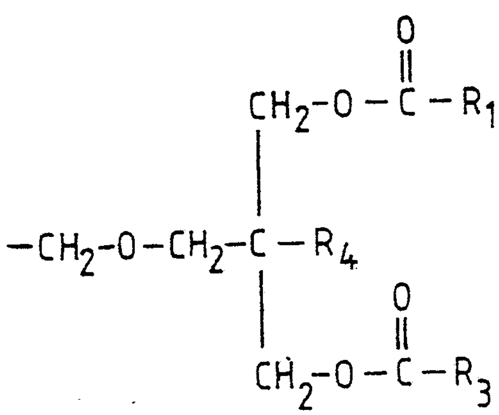

(III)

7. Use of the esters purified by the process according to any preceding Claim in the fields of oleodynamic fluids, lubricating oils, cutting fluids, insulating oils (dielectric oils) and drilling muds.